# EUROPEAN PATENT SPECIFICATION

(11) **EP 0 706 794 B1**
(45) Date of publication and mention of the grant of the patent: **12.12.2001**
(21) Application number: 95307146.1
(22) Date of filing: 10.10.1995
(51) Int. Cl.: A61K 31/425, A61K 38/55, A61K 9/16

(54) **Anti-aids pharmaceutical preparations and processes for the production thereof**
AIDS-hemmende Arzneizubereitung und Verfahren
Composition pharmaceutique anti-SIDA et procédé de préparation

(30) Priority: 14.10.1994 JP 27610294
(43) Date of publication of application: 17.04.1996
(73) Proprietor: Japan Energy Corporation, Tokyo-to 105-8407 (JP)
(72) Inventor: Muto, Akihiro, Toda-shi, Saitama-ken (JP); Takeuchi, Naoto, Toda-shi, Saitama-ken (JP); Fujisawa, Naoki, Toda-shi, Saitama-ken (JP)
(74) Representative: Davies, Jonathan Mark

(56) References cited:
- EP-A- 0 490 667
- EP-A- 0 498 680
- EP-A- 0 587 311
- WO-A-93/13066

## Description

This invention relates to anti-AIDS pharmaceutical preparations suitable for oral administration and processes for the production thereof. Particularly, this invention relates to the anti-AIDS pharmaceutical preparations suitable for oral administration comprising human immunodeficiency virus (HIV) protease inhibitor which inhibits the enzymic activity of HIV derived protease as an active ingredient and processes for the production thereof.

### Background of the Invention

A pathogenic virus of AIDS, human immunodeficiency virus (HIV), first produces its precursor protein as a complex protein in the proliferation process. Said complex protein is cleaved by a viral derived protease (HIV protease) to fractions having particular sizes and exhibit functions of respective fractions. Therefore, the HIV protease inhibitor blocks the formation and maturity of infectious viral particles by the inhibition of enzymic activity of HIV protease and exhibits the antiviral activity. Several types of HIV protease inhibitors have been reported including synthetic peptides called substrate transition state pseudo-peptides (see T. Robins, J. Plattner, J. Acquir. Immun. Defic. Syndr., 6, 162 (1993)), The present inventors found a new series of synthetic peptides of the substrate transition state pseudo-peptides having 3-amino-2-hydroxy-4-phenylbutanoic acid in their fundamental structure, which strongly inhibit the HIV protease activity and will be useful as anti-AIDS medicine. Thus, the inventors proposed them as HIV protease inhibitors (see Japanese Published Un-examined Patent Application No. 170,722 (1993)). HIV protease inhibitors, particularly substrate transition state pseudo-peptides, are highly expected as anti-AIDS agents of the following generation after reverse transferase inhibitors of nucleic acid derivatives which have been clinically used such as AZT, dideoxycytidine (DDC) and dideoxyinosine (DDI), and now under clinical trials and investigations.

However, these synthetic peptides are reported to have drawbacks such as difficult solubility in water or low oral absorption rate (see Hiroaki Mitsuya, Science, 64(7), 462-470 (1994)). That is, pharmaceutical preparations for oral administration composed of said peptide which are difficultly soluble in water and prepared by mixing with conventional fillers or additives are difficult to be absorbed from digestive tract, which leads to their low bioavailability. Particularly, anti-AIDS medicines are generally successively administered for a long period of time, thus, their preparations for oral administration with improved bioavailability have been desired.

Generally, preparation of fine powder to increase specific surface area or conversion into non-crystalline form is applied to improve oral absorption rate of difficultly soluble medicines. However, no general method to prepare non-crystalline forms of difficultly soluble medicines or oral preparations with desired disintegration and dissolution rates has been known. In other words, improved disintegration and dissolution rates, which largely depend on the characteristic features of individual medicine, to give anti-AIDS pharmaceutical preparations with satisfactory oral absorbability have been desired.

The inventors have been investigating a method to prepare a non-crystalline form of a series of HIV protease inhibitive synthetic peptides (for this series of peptides see Japanese Published Unexamined Patent Application No. 170,722 (1993)) and found to give non-crystalline fine powder of compounds shown by the general formula (I) by dissolving them in a solvent followed by spray drying. However, the resultant fine powder has insufficient water-wettability and low dissolution rate. Furthermore, in capsule preparations made from the non-crystalline fine powder, the fine powder coagulate each other by contacting with water and could not provide improved disintegration and solubility rates for oral use.

The present inventors further investigated on the bases of above mentioned findings and found to give oral preparations with excellent disintegration, dissolution and high oral absorption rates by coating solid composition composed of synthetic peptide in non-crystalline fine powder form and a pharmacologically acceptable binder on pharmaceutically acceptable core particles and accomplished the present invention.

### Summary of the Invention

One object of the present invention is to provide anti-AIDS pharmaceutical preparations containing HIV protease inhibitive synthetic peptide compound which can be easily absorbed from digestive tract with high bioavailability and suitable for oral administration as an active ingredient.

The anti-AIDS pharmaceutical preparations of the present invention are prepared by coating core particles with solid compositions composed of (i) a non-crystalline form of a compounds shown by general formula (I) or pharmacologically acceptable salts thereof and (ii) a pharmacologically acceptable binder selected from hydroxypropylmethylcellulose, hydroxypropylcellulose, polyvinylpyrrolidone, polyethylene glycol and hydroxypropylmethylcellulose phtalate. 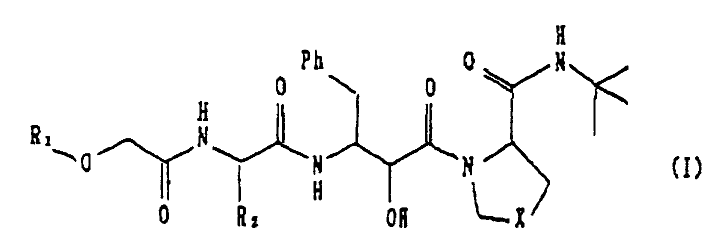 (wherein R₁ represents 5-isoquinolyl group or 3-pyridyl group, R₂ represents methylthiomethyl group or isopropyl group, and X represents sulfur atom or methylene group).

The other object of the present invention is to provide processes for the production of anti-AIDS pharmaceutical preparations prepared by spraying pharmacologically acceptable core particles with a solution or suspension composed of (i) a compound shown by general formula (I) or pharmacologically acceptable salts thereof and (ii) a pharmacologically acceptable binder selected from hydroxypropylmethylcellulose, hydroxypropylcellulose, polyvinylpyrrolidone, polyethylene glycol and hydroxypropylmethylcellulose phtalate and drying, thus coating said core particles with solid compositions composed of non-crystalline form of said compound or a salt thereof and said pharmaceutically acceptable binder.

### Brief Description of Drawings

Fig. 1 shows a diffraction pattern of powder X-ray diffraction of fine particles of compound (A) obtained by Reference example 1.
Fig. 2 shows a diffraction pattern of powder X-ray diffraction of micro-crystalline compound (A) obtained by Comparative example 1.
Fig. 3 shows a powder X-ray diffraction pattern of anti-AIDS granular preparation obtained by Example 4.
Fig. 4 shows a diffraction pattern of powder X-ray diffraction of a mixture prepared by mixing non-crystalline fine powder of said compound (A) prepared by Reference example 1, HPMC and Celphere at the same ratio of anti-AIDS granular preparation according to Example 4.
Fig. 5 shows a diffraction pattern of powder X-ray diffraction of core particles of Celphere (crystalline cellulose).
Fig. 6 shows a diffraction pattern of powder X-ray diffraction of a mixture prepared by mixing micro-crystalline powder of said compound (A) prepared by Comparative example 1, HPMC and Celphere at the same ratio of anti-AIDS granular preparation according to Example 4.
Fig. 7 shows an increased rate of dissolution of anti-AIDS granular preparation obtained by Examples 1-4 plotted to the progress of time.

### Detailed Description of the Invention and preferred Embodiment

Compounds shown by above mentioned general formula (I) of the present invention, that is HIV protease inhibitive synthetic peptides include, for example, N-tert-butyl-3-[2-hydroxy-3-[2-(5-isoquinolyloxyacetyl)amino-3-methylthiopropanoyl]amino-4-phenylbutanoyl]-1,3-thiazolidine-4-carboxamide, N-tert-butyl-1-[2-hydroxy-3-[2-(5-isoquinolyloxyacetyl)amino-3-methylthiopropanoyl]amino-4-phenylbutanoyl]pyrrolidine-2-carboxamide, N-tert-butyl-3-[2-hydroxy-3-[2-(5-isoquinolyloxyacetyl)amino-3-methylbutanoyl]amino-4-phenylbutanoyl]-1,3-thiazolidine-4-carboxamide, N-tert-butyl-1-[2-hydroxy-3-(2-(5-isoquinolyloxyacetyl)amino-3-methylbutanoyl]amino-4-phenylbutanoyl]pyrrolidine-2-carboxamide, N-tert-butyl-3-[2-hydroxy-3-[2-(3-pyridyloxyacetyl)amino-3-methylpropanoyl]amino-4-phenylbutanoyl]-1,3-thiazolidine-4-carboxamide, N-tert-butyl-3-[2-hydroxy-3-[2-(3-pyridyloxyacetyl)amino-3-methylthiopropanoyl]amino-4-phenylbutanoyl]pyrrolidine-4-carboxamide, N-tert-butyl-3-[2-hydroxy-3-[2-(3-pyridyloxyacetyl)amino-3-methylbutanoyl]-amino-4-phenylbutanoyl]-1,3-thiazolidine-4-carboxamide and N-tert-butyl-1-[2-hydroxy-3-[2-(3-pyridyloxyacetyl)amino-3-methylbutanoyl]amino-4-phenylbutanoyl]pyrrolidine-2-carboxamide.

These compounds all exhibit specific and high HIV protease inhibitory activity and are useful as anti-AIDS agents. Therefore, these compounds may be contained in the anti-AIDS pharmaceutical compositions of the present invention as an active ingredient. Particular compounds having basic structure of (2S, 3S)amino-4-phenylbutanoyl group, for example, (R)-N-tert-butyl-3-[(2S, 3S)-2-hydroxy-3-[(R)-2-(5-isoquinolyloxyacetyl)amino-3-methylthiopropanoyl]amino-4-phenylbutanoyl]-1,3-thiazolidine-4-carboxamide, (S)-N-tert-butyl-1-[(2S, 3S)-2-hydroxy-3-[(S)-2-(5-isoquinolyloxyacetyl)amino-3-methylbutanoyl]amino-4-phenylbutanoyl]pyrrolidine-2-carboxamide, and (R)-N-tert-butyl-3-[(2S, 3S)-2-hydroxy-3-[(S)-2-(3-pyridyloxyacetyl)-amino-3-methylbutanoyl]amino-4-phenylbutanoyl]-1,3-thiazolidine-4-carboxamide exhibit very potent inhibition of HIV protease activity and especially (R)-N-tert-butyl-3-[(2S, 3S)-2-hydroxy-3-[(R)-2-(5-isoquinolyloxy-acety])amino-3-methylthiopropanoyl]amino-4-phenylbutanoyl]-1,3-thiazolidine-4-carboxamide is more preferable. These synthetic peptides may be used in the form of pharmacologically acceptable salts.

The pharmacologically acceptable core particles used in the present invention are solid at the temperature of oral administration and also at the temperature of coating process. In addition, these solid core particles having no or very low solubility in a solvent used for wet coating method are preferably used when the process for the production is conducted by vet coating methods. Furthermore, said core particles having hardness that can endure without abrasion or disintegration during the coating process are preferably used. These particles having polyhedral forms that can be regarded as spherical or ball 1 shaped are preferably used for the present invention.

The core particles are suitably selected from raw materials according to the types of above mentioned peptides, binders, and methods and conditions of coating, and include such as lactose, sucrose, corn starch, anhydrous silicic acid, and crystalline cellulose, particularly spherical crystalline cellulose is preferably used. For example, spherical crystalline cellulose is particularly preferred core for wet coating method using alcohol or an aqueous alcohol. The diameters of ball shaped core particles are suitably determined according to the types of active ingredient of synthetic peptide to be coated, but the larger the diameter the less the specific surface area, thus small diameters are generally preferred. For example, diameters of 100-700 µm, particularly 150-300 µm are suitably selected for synthetic peptides shown above. If too smaller amount of said synthetic peptide is used per particle, then the greater total weight or volume of the anti-AIDS pharmaceutical preparation will be unnecessarily required to obtain the desired dose. Thus, 0.5-10 parts by weight, preferably 1-5 parts by weight of core particles are suitably selected for one part by weight of the peptide compounds.

The pharmacologically acceptable binders used in the present invention will be compounds that can be dissolved or dispersed in a solvent used in the wet coating methods and easily wettable, dispersible or soluble in water. For the synthetic peptides shown above, hydrophilic hydroxypropylmethylcellulose, hydroxypropylcellulose, polyvinylpyrrolidone, polyethylene glycol, hydroxypropylmethylcellulose phthalate and so forth are suitably used. Particularly, hydroxypropylmethylcellulose is preferred. The weight ratio of the mixture of the binders and synthetic peptides are suitably determined according to the types of active ingredient synthetic peptides to be coated, and too small amount of binders results in insufficient binding property for the coating of core particles and too large amount of binders leads to adhesion of the obtained preparation to result in a formation of aggregation. Thus, 0.5-10 parts bY weight, preferably 1-8, more preferably 3-6 parts by weight of binders are suitably selected for 10 parts by weight of the synthetic peptide compounds. For example, combinations of 10 parts by weight of the synthetic peptides illustrated above and 3-6 parts by weight of hydroxypropylmethylcellulose are more preferably selected.

In the process for the production of present invention, fluidized bed coating method is suitably selected for the spray dry coating of liquid or suspension compositions composed of compounds shown by the above mentioned general formula (I) or pharmacologically acceptable salts thereof and a pharmacologically acceptable binder. That is, the synthetic peptide compound as an active ingredient and the binder are mixed at a predetermined weight ratio and the resultant mixture is used to prepare a solution or a suspension in an organic solvent or a mixture of an organic solvent and water. The core particles are fluidized in hot air flow and the coating liquid is sprayed and dried to form the coating layer. An apparatus for the coating of granular preparations can be diverted for the fluidized bed coating and, for example, a tumbling fluidized bed coating apparatus is conveniently used.

The coating liquid may be prepared using any solvent such as an organic solvent or a mixture of an organic solvent and water, which can dissolve or disperse the synthetic peptide and the binder, and can volatile or transpire under temperatures used for the spray coating. For example, lower alcohols such as methanol and ethanol or their mixtures with water are more preferable to easily provide fine powder of above mentioned non-crystalline synthetic peptides. The concentration of the coating liquid can be determined according to the capacity of the coating apparatus and viscosity of the liquid. For example, one part by weight of the synthetic peptide is mixed with 5-50, preferably 10-20 parts by weight of mixtures of methanol or ethanol with added no more than 50, preferably 10-20 weight percent of water. That is, mixtures of a lower alcohol such as ethanol and a small amount of water provide more uniformly dispersed coating layer composed of improved fine and non-crystalline synthetic peptide and the binders with better water solubility. For the preparation of suspensions as coating liquids, the synthetic peptides are made non-crystalline form in advance and then dispersed in the solvent, particularly very fine non-crystalline powder is more preferable for the preparation of uniformly dispersed suspensions. In general, suspensions are more preferably prepared by dissolving most of the synthetic peptides in a solvent and the remaining a small amount of undissolved non-crystalline peptide powder is dispersed.

The anti-AIDS pharmaceutical preparations prepared by the processes of the present invention provide complex particles or fine granules of solid cores coated with a uniformly dispersed coating layer composed of the non-crystalline synthetic peptide and the binder. Practically, fine non-crystalline form of the synthetic peptide compound and the binder in the coating liquid are uniformly dispersed and spray dried to give complex granules or fine granules coated with the uniformly dispersed layer. The resultant complex particles of anti-AIDS pharmaceutical preparations may be further coated with pharmacologically acceptable additives free from the synthetic peptide for the prevention of charging of static electricity or moisture, improvement of fluidity or taste (e.g. sugar coating), light shielding, and modulation of dissolution rate.

The anti-AIDS pharmaceutical preparations of the present invention take forms of particles or fine granules of complex particles, and can be orally administered as it is. The preparations may be further filled in oral capsules such as gelatin and HPMC, tableted using conventional tableting machine by addition and mixing with conventional fillers such as lactose or starch as a diluent, hydroxypropylcellulose as a binder, crystalline cellulose as a disintegrating agent, magnesium stearate or talc as a lubricant. The obtained plain tablets may further be coated with enteric coating materials such as hydroxypropylmethylcellulose phthalate to give enteric coating tablets, or with long lasting coating agent such as ethylcellulose to give sustained release tablets. In addition, granules or fine granules of anti-AIDS pharmaceutical preparations of the present invention may further be enteric or sustained release coated to give enteric coated preparations or long lasting granules or fine granules. These coated granules or fine granules may be filled in capsules or tableted. In addition, the content of compounds shown by general formula (I) in capsules or tablets for oral administration may be suitably determined according to the symptoms and the aims of administration.

The anti-AIDS pharmaceutical preparations of the present invention have a coating layer homogeneously containing non-crystalline peptide compound as an active ingredient and a binder on the core particles. Thus, the preparations of the present invention have excellent disintegration and dissolution properties in water and are easily absorbed from the digestive tract to give high bioavailability. Therefore, the anti-AIDS pharmaceutical preparations of the present invention are suitable for oral administration. Particularly, the high bioavailability markedly reduces the required amount of oral doses to obtain the desired blood concentrations of the active ingredient peptides. Or the total weight or volume of the final form of the anti-AIDS preparation can be reduced, and the preparations of the present invention provide particularly preferred effect as oral preparations. The processes for the production of the anti-AIDS pharmaceutical preparations of the present invention produces a large amount of the anti-AIDS pharmaceutical preparations with an uniform quality by a simple procedure.

The process for the production of anti-AIDS pharmaceutical preparations of the present invention will be explained by examples. Also, the examples show high bioavailability of the anti-AIDS pharmaceutical preparations of the present invention.

The following examples are enumerated to practically explain the present invention and do not restrict the scope of the present invention.

### [Example 1]

### Preparation of anti-AIDS pharmaceutical preparations

A granular preparation was prepared by coating core particles with a composition containing a non-crystalline synthetic peptide compound and a binder by the following process. Except otherwise stated, compounds approved by Japanese Pharmacopoeia (JP) are generally used in the following processes.

### Process (1-1): Preparation of a coating solution.

Crystals of one of peptide compound shown by general formula (I), (R)-N-tert-butyl-3-[(2S, 3S)-2-hydroxy-3-[(R)-2-(5-isoquinolyloxyacetyl)amino-3-methylthiopropanoyl]-amino-4-phenylbutanoyl]-1,3-thiazolidine-4-carboxamide (hereinafter referred as compound (A)) were used as raw materials. Hydroxypropylmethylcellulose 2910 (hereinafter referred as HPMC) was used as a binder. A mixture of 837 g of methanol (Japanese Industrial Standards (JIS), Extra pure) and 93 g of purified water was used as a solvent. In 930 g of the mixed solvent, 50 g of compound (A) and 20 g of HPMC were dissolved and made 1,000 g in total to make a coating solution.

### Process (1-2): Preparation of granular preparation by coating

Spherical crystalline cellulose was used as core particles. Commercial spherical cellulose, Celphere CP203 (Asahi Chemical Industry Co., Ltd. Japan, hereinafter referred as Celphere) having genuine spherical form, particle sizes of 150-300 µm, was used. In a rolling fluidized bed coating apparatus (trade name: Multiplex, product of Powrex Co.. Ltd.,Japan), 500 g of Celphere was floated at a air supply temperature of 55°C and air flow rate of 40 m³/hr, and the coating solution obtained by the process (1-1) was spray-dried to form a coating layer on the surface of Celphere.

After drying, the prepared granular preparation was recovered and sieved with a 42 mesh sieve. The amount of granules that had not pass through the sieve was 1%. These un-sieved granules formed aggregation of plural particles and the occurrence rate was estimated as 1%. The average content of compound (A) in the coated particles of anti-AIDS granules, without formation of aggregation was estimated by dissolving the compound (A) in the granules in a liquid and the concentration of compound (A) was quantitatively determined by ultraviolet absorption method. Assuming that the total amount of compound (A) in the coating solution was coated and the amount of compound (A) to be contained in the granules was made 100%, then the average content of compound (A) in the granules was 98%, indicating the coating rate of 98%.

### [Example 2]

According to the preparation procedure of Example 1 shown above, an anti-AIDS pharmaceutical, preparation was prepared by the combination of the following raw materials. A mixture of 3,348 g of methanol and 372 g of purified water was used for the coating solution. In the mixture, 200 g of compound (A) and 80 g of HPMC were dissolved to prepare the coating solution. The coating solution was coated on 500 g of Celphere CP203 by fluidized bed coating to give the granular preparation.

In the present Example, the occurrence rate of un-sieved aggregation was estimated as 2.4%. The average content of compound (A) in the anti-AIDS granular preparation was analyzed according to the above mentioned analytical procedure of Example 1 and the coating rate was estimated as 95%.

### [Example 3]

According to the preparation procedure of Example 1 shown above, an anti-AIDS pharmaceutical preparation was prepared by the combination of the following raw materials. A mixture of 3,276 g of methanol and 364 g of purified water was used for the coating solution. In the mixture, 200 g of compound (A) and 160 g of HPMC were dissolved to prepare the coating solution. The coating solution was coated on 500 g of Celphere CP203 by fluidized bed coating to give the granular preparation.

In the present Example, the occurrence rate of un-sieved aggregation was estimated as 10.4%. The average content of compound (A) in the anti-AIDS granular preparation was analyzed according to the above mentioned analytical procedure of Example 1 and the coating rate was estimated as 97%.

### [Example 4]

According to the preparation procedure of Example 1 shown above, an anti-AIDS pharmaceutical preparation was prepared by the combination of the following raw materials, A mixture of 5,025 g of ethanol, JP and 942 g of purified water was used for the coating solution. In the mixture, 500 g of compound (A) and 200 g of HPMC were dissolved to prepare the coating solution. The coating solution was coated on 500 g of Celphere CP203 by fluidized bed coating to give the granular preparation.

In the present Example, the resultant granules were sieved with a 35 mesh sieve and the occurrence rate of un-sieved aggregation was estimated as 3.3%. The average content of compound (A) in the anti-AIDS granular preparation was analyzed according to the above mentioned analytical procedure of Example 1 and the coating rate was estimated as 92%.

### [Reference example 1]

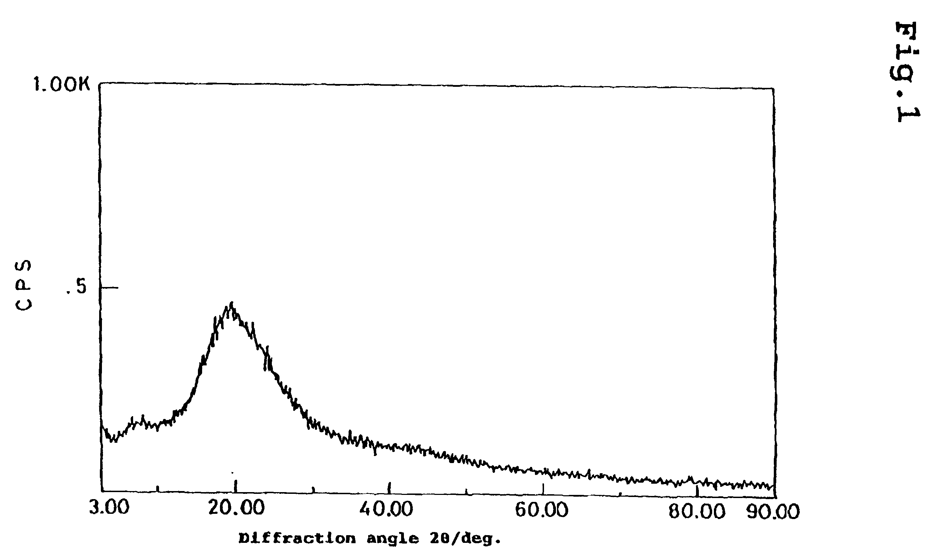

In 190 g of methanol, 10 g of compound (A) used as a raw material in the above mentioned Example 1 was dissolved. The solution was spray-dried in the absence of the core particles to give fine powder of compound (A). The fine powder was analyzed with powder X-ray diffraction and a diffraction pattern shown by Fig. 1 was obtained. No characteristic sharp diffraction pattern of highly crystalline sample was observed indicating very poor crystallinity, that is non-crystalline compound. The non-crystalline fine powder was made as the Reference example 1 in the following various Test examples.

### [Comparative example 1]

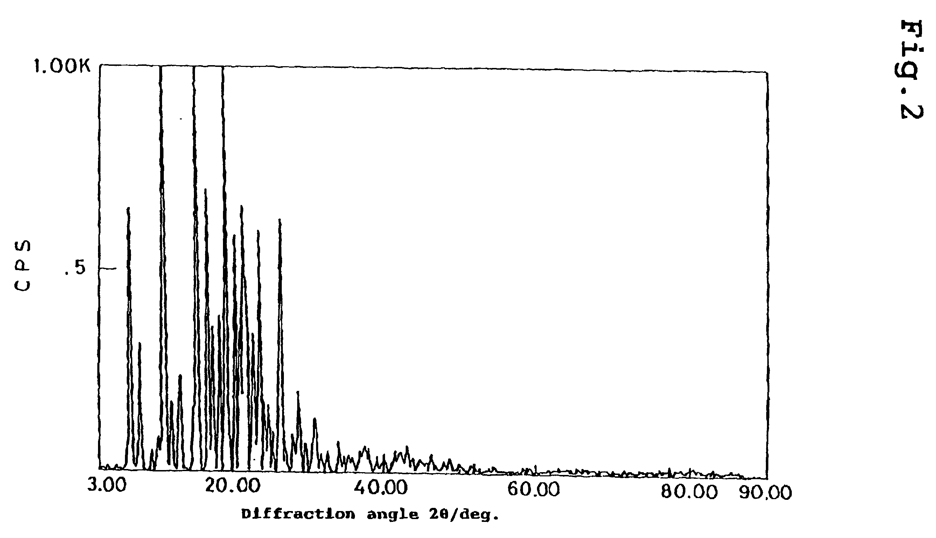

Micro-crystals of compound (A) used as raw materials in the above mentioned Examples 1-4 were used in the following Test examples as the Comparative example 1. The micro-crystals analyzed by powder X-ray diffraction gave a sharp diffraction pattern shown in Fig. 2 indicating their high crystallinity.

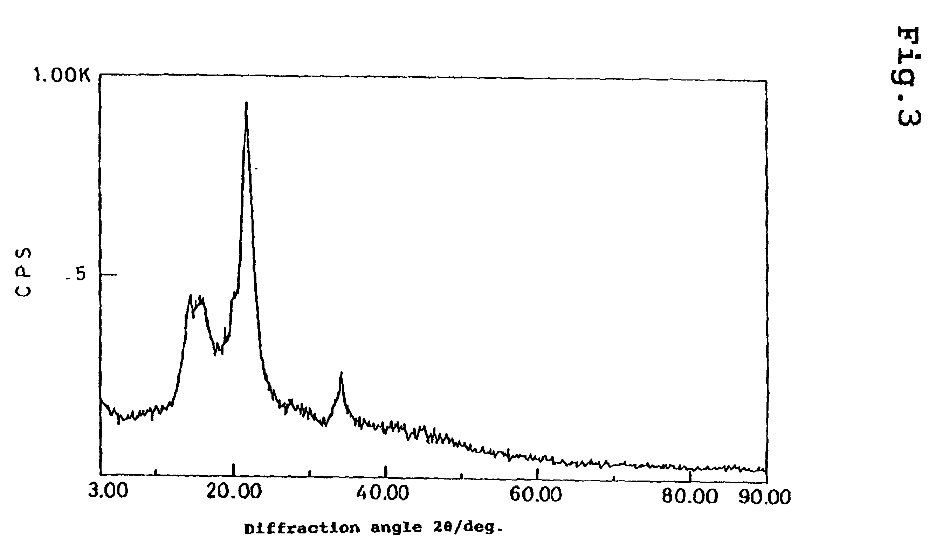

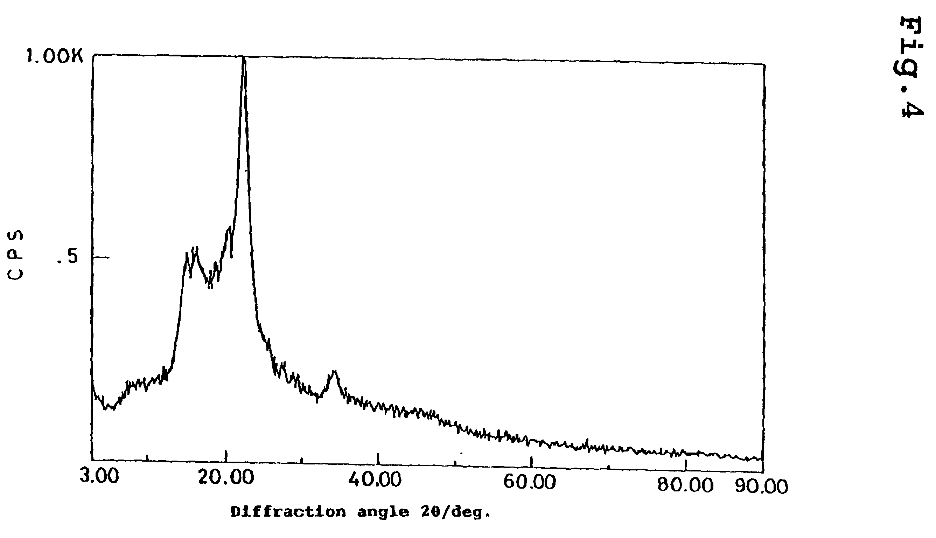

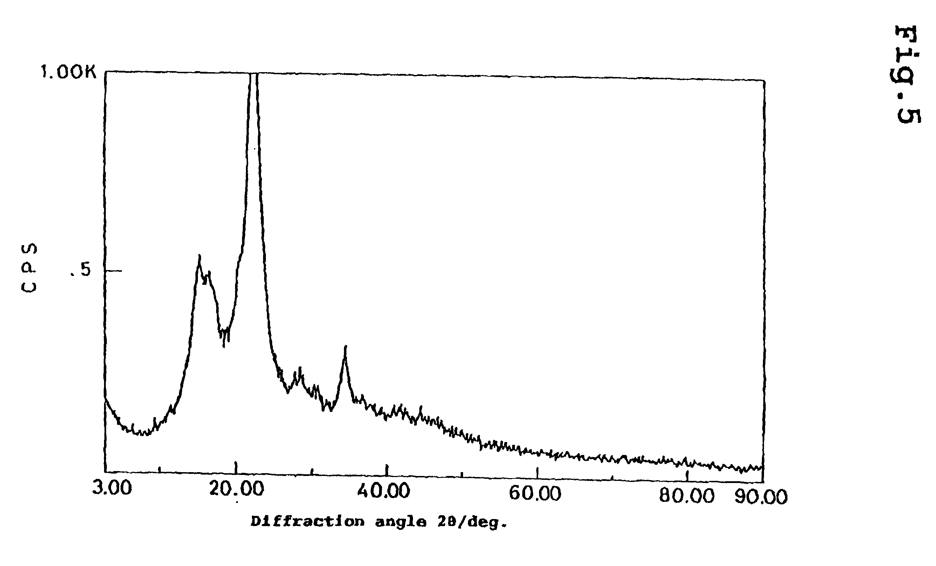

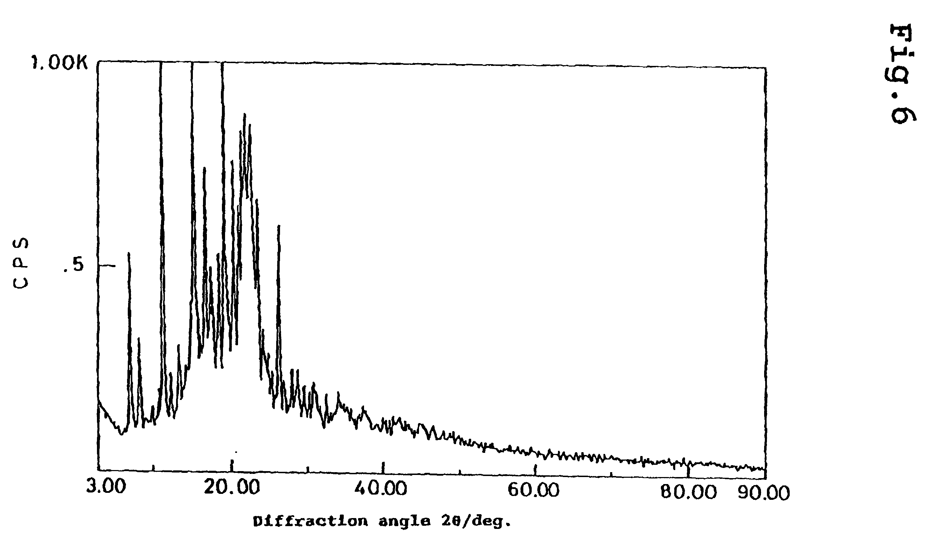

On the other hand, determination of X-ray diffraction pattern of anti-AIDS granules obtained by Example 4 gave a diffraction pattern shown in Fig. 3. Fine powder of non-crystalline compound (A) obtained by Reference example 1, HPMC and Celphere were mixed at the same ratio with those contained in the anti-AIDS granules obtained by Example 4. The powder mixture was determined by powder X-ray diffraction and a refraction pattern shown in Fig. 4 was obtained. Comparison of the two diffraction patterns of Fig. 3 and 4 showed very close patterns. Determination of powder X-ray diffraction of core particle Celphere (crystalline cellulose) gave a refraction pattern shown in Fig. 5. That is, the patterns of both Fig. 3 and 4 correspond to the added patterns of sole core particles of Celphere shown in Fig. 5 and fine powders of non-crystalline compound (A) obtained by Reference example 1 shown in Fig. 1, respectively. Furthermore, determination of powder X-ray diffraction pattern of a mixture of fine crystalline powder of compound (A) obtained by Comparative example 1. HPMC and Celphere at the same ratio contained in the anti-AIDS granules obtained by Example 4 gave a refraction pattern shown in Fig. 6. The refraction pattern of Fig. 6 corresponds to a combinated refraction patterns of core particles solely prepared of Celphere shown in Fig. 5 and a characteristic sharp refraction pattern of micro-crystals shown by Fig. 2. Above mentioned comparisons confirm the presence of non-crystalline fine powder of compound (A) in the anti-AIDS granules obtained by Example 4. In addition, determination of powder X-ray diffraction patterns of anti-AIDS granules obtained by Examples 1-3 gave very close refraction patterns with that shown in Fig. 3 confirming the fine powder coating of non-crystalline compound (A).

### [Test example 1]

The dissolution and release characteristics of compound (A) in the anti-AIDS granular preparations obtained by the above mentioned Examples 1-4 were examined by the following method.

Method for evaluation of dissolution characteristics.

The test t samples of anti-AIDS granular preparations were prepared to contain 150 mg of total amount of compound (A). The dissolution test was achieved according to the paddle method in JP using 900 ml of purified water, at a test temperature of 37°C and paddle rotation rate of 100 rpm. The total amount of dissolved compound (A) was calculated from the determined value by ultraviolet ray absorption method.

The dissolution rate of compound (A) after 2 hrs. from anti-AIDS granular preparations obtained by Examples 1-4 are shown in Table 1. The dissolution rate was estimated by dividing the total weight of dissolved compound (A) by the total content of 150 mg of compound (A) in the test sample, The dissolution and release characteristics of compound (A) from non-crystalline fine powder obtained by Reference example 1 and crystalline fine powder obtained by Comparative example 1 were similarly evaluated and the dissolution rate after 2 hrs. are shown in Table 1 for the comparison.

**[Table 1]**

| Test sample | Dissolution rate (%) |
|---|---|
| Example 1 | 56 |
| Example 2 | 48 |
| Example 3 | 48 |
| Example 4 | 53 |
| Reference example 1 | 18 |
| Comparative example 1 | 5 |

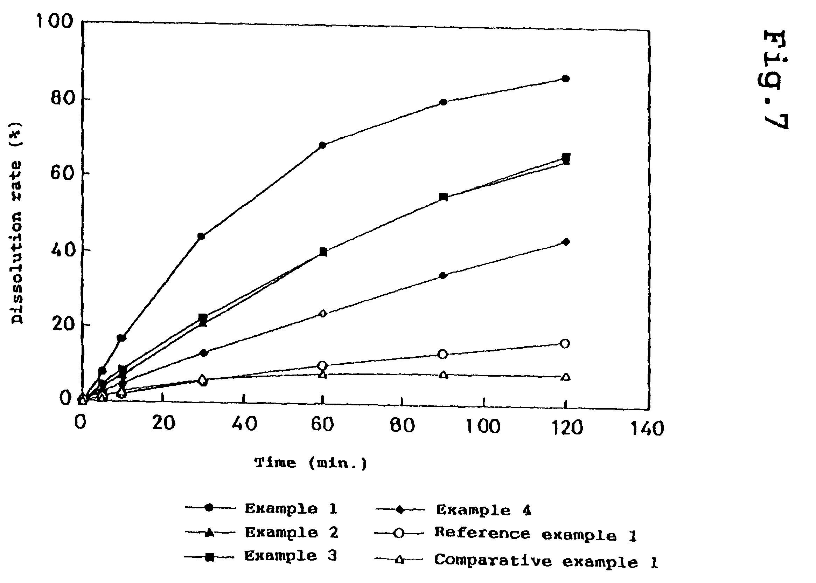

In addition, test sample of anti-AIDS granular preparation was prepared according to the above mentioned method to give total content of 75 mg of compound (A) and the dissolution and release characteristic was evaluated according to the method shown above. The dissolution rate of compound (A) after 2 hrs. from anti-AIDS granular preparations obtained by Examples 1-4 are shown in Table 2. The dissolution rate was estimated by dividing the total weight of dissolved compound (A) by the total content of 75 mg of compound (A) in the test samples. The dissolution and release characteristics of compound (A) from non-crystalline fine powder obtained by Reference example 1 and crystalline fine powder obtained by Comparative example 1 were similarly evaluated and the dissolution rate after 2 hrs. are shown in Table 2 for the comparison. The increased dissolution rate of each test sample was plotted against the progress of time and the results are shown in Fig. 7.

**[Table 2]**

| Test sample | Dissolution rate (%) |
|---|---|
| Example 1 | 87 |
| Example 2 | 65 |
| Example 3 | 66 |
| Example 4 | 44 |
| Reference example 1 | 17 |
| Comparative example 1 | 8 |

Above results indicate that all granular preparations of the present invention have far superior dissolution rate than crystalline fine powder of Comparative example 1. Furthermore, comparison with that of non-crystalline fine powder of Reference example 1 clearly showed superior solubility of the granular preparation of the present invention with improved dissolution rate of the granular preparations of the present invention obtained by coating of the core particles with non-crystalline compound (A) and a binder.

### [Test example 2]

Capsule preparations evenly filled with anti-AIDS granular preparations each prepared by the above mentioned Examples 1-4 in No. 1 hard gelatin capsules of 0.47 ml volume were prepared. The disintegration test was carried out according to the method in JP using purified test water and at 37°C. The determined disintegration time are shown in Table 3. In addition, similar capsule preparations were prepared for the fine powder of non-crystalline compound (A) of Reference example 1 and crystalline fine powder of Comparative example 1, and the disintegration test was performed. The obtained disintegration time are comparatively shown in Table 3.

**[Table 3]**

| Test capsule preparation | Disintegration time (min.) |
|---|---|
| Example 1 | 5.1 ± 1.8 |
| Example 2 | 2.3 ± 0.4 |
| Example 3 | 15.6 ± 4.4 |
| Example 4 | 4.2 ± 1.3 |
| Reference example 1 | 30 or over |
| Comparative example 1 | 5.0 ± 2.0 |

Capsule preparations filled with granular preparations of Examples 1-4 exhibited far shorter distintegration time than those of non-crystalline fine powder of Reference example 1. In addition, capsule preparations filled with granular preparations of Example 1, 2 and 4 showed similar or shorter disintegration time than those filled with fine crystalline powder of Comparative example 1, When the improved dissolution rate and the disintegration rate are considered together, capsule preparations prepared of granules of Examples 1-4 exhibited the release of compound (A) at ratios of far greater amount than those of Comparative example 1 and Reference example 1.

### [Test example 3]

In hard gelatin capsules, four types of anti-AIDS granular preparations prepared by the above mentioned Examples 1-4 were filled, respectively, each containing 150 mg of compound (A). These four types of capsule preparations were orally given each to fasted male beagle dog group having three dogs and body weight of about 10 kg. After the administration, blood was drawn periodically from the test animals and the plasma concentration of compound (A) was quantitatively determined by high performance liquid chromatography (HPLC). In the control, compound (A) was intravenously administered at a dose of 15 mg/kg and the blood was drawn from each of the tested animals periodically to determine the plasma concentration of compound (A).

In the above control, area under the plasma concentration curve (AUC) of compound (A) after the administration was used as the standard, that is 100% of bioavailability. After oral administration, the AUC of compound (A) was converted on the basis of body weight and compared with those of control to give the bioavailability of compound (A). The calculated bioavailabilities of four capsule preparations made from granular preparations of Examples 1-4, respectively, are shown in Table 4. A capsule preparation filled with fine powder of compound (A) of Comparative example 1 was similarly and orally administered to fasted animals and the calculated bioavailability is shown in Table 4 for the comparison. The bioavailability is shown by average values with standard deviations.

**[Table 4]**

| Test capsule preparation | Bioavailability (%) |
|---|---|
| Example 1 | 41.4 ± 6.1 |
| Example 2 | 31.4 ± 19.2 |
| Example 3 | 41.0 ± 14.8 |
| Example 4 | 32.7± 7.9 |
| Comparative example 1 | 12.4 ± 1.5 |

In hard gelatin capsules, three types of anti-AIDS granular preparations prepared by the above mentioned Examples 1-3 were filled, respectively, each containing 150 mg of compound (A). These three types of capsule preparations were orally given to the other male beagle dog groups each having three dogs and body weight of about 10 kg in a similar manner and the oral bioavailability was evaluated where the control data measured using only one animal were used as the standard.

**[Table 5]**

| Test capsule preparation | Bioavailability (%) |
|---|---|
| Example 1 | 61.1 |
| Example 2 | 63.8 |
| Example 3 | 51.6 |
| Comparative example 1 | 28.7 |

Comparison of bioavailabilities of above mentioned capsule preparations by oral administration revealed that four type capsule preparations made from granules of Examples 1-4 exhibit far superior bioavailability than that of made from fine powder of Comparative example 1. The evaluated results of bioavailability of orally administered capsule preparations shown in Tables 4 and 5 show a systematic deviation, which is considered to reflect the individual differences in the test animals used, especially for the control, but four capsule preparations made from granules of Example 1-4 showed about 2-fold bioavailability to that of capsule preparation filled with fine powders of Comparative example 1.

## Claims

1. Anti-AIDS pharmaceutical preparations comprising pharmaceutically acceptable core particles having coated thereon a solid composition composed of
(i) a non-crystalline compound of formula (I) shown below or a pharmacologically acceptable salt thereof and
(ii) a pharmacologically acceptable binder selected from hydroxypropylmethylcellulose, hydroxypropylcellulose, polyvinylpyrrolidone, polyethylene glycol and hydroxypropylmethylcellulose phtalate 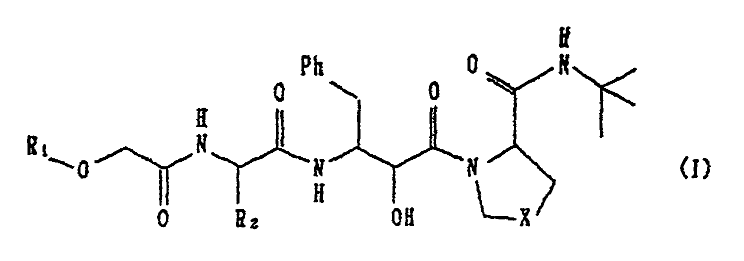
wherein R₁ represents 5-isoquinolyl group or 3-pyridyl group, R₂ represents methylthiomethyl group or isopropyl group and X represents sulfur atom or methylene group and wherein the solid composition contains 0.5-10 parts by weight binder for 10 parts by weight compound of formula (I) or salt thereof.

2. The anti-AIDS pharmaceutical preparations according to Claim 1, wherein the core particles are particles having diameters of 100-700 µ m.

3. The anti-AIDS pharmaceutical preparations according to Claim 1 or 2, wherein the core particles are made of crystalline cellulose.

4. The anti-AIDS pharmaceutical preparations according to Claims 1-3, wherein the binder is hydroxypropylmethylcellulose.

5. The anti-AIDS agent according to Claims 1-4, wherein the compound shown by general formula (I) has a 3-(2S, 3S)amino-4-phenylbutanoyl-1,3-thiazoline-4-carboxamide structure.

6. The anti-AIDS agent according to Claim 5, wherein the compound shown by general formula (I) is (R)-N-tert-butyl-3-[(2S, 3S)-2-hydroxy-3-[(R)-2-(5-isoquinolyloxyacetyl) amino-3-methylthiopropanoyl]amino-4-phenylbutanoyl]-1,3-thiazolidine-4-carboxamide.

7. A process for the production of anti-AIDS pharmaceutical preparations comprising spray drying a solution or a suspension containing
(i) compounds shown by general formula (I) or pharmacologically acceptable salts thereof,
(ii) a pharmacologically acceptable binder selected from hydroxypropylmethylcellulose, hydroxypropylcellulose, polyvinylpyrrolidone, polyethylene glycol and hydroxypropylmethylcellulose phtalate and
(iii) lower alcohols or a lower alcohols/water mixture as a solvent
(iv) wherein the coating solution or suspension contains 10 parts by weight of the compound shown by general formula (I) and 0.5-10 parts by weight of the binder,
onto pharmaceutically acceptable core particles such that the particles become uniformly coated with non-crystalline forms of the compounds of formula (I) or salts thereof and the binder; and drying the coated particles.

8. A process for the production of anti-AIDS pharmaceutical preparations including the step of further coating pharmaceutical preparations prepared by a process according to Claim 7 with an enteric coating agent.

9. The process for the production of anti-AIDS pharmaceutical preparations according to claim 8, wherein the enteric coating agent is hydroxypropylmethylcellulose phthalate.

## Patentansprüche

1. Pharmazeutische Anti-AIDS-Präparate, umfassend pharmazeutisch akzeptable Kernpartikel, die mit einer festen Zusammensetzung beschichtet sind, umfassend
(i) eine nichtkristalline Verbindung der nachfolgend dargestellten Formel (1) oder ein pharmakologisch akzeptables Salz davon und
(ii) ein pharmakologisch akzeptables Bindemittel, ausgewählt aus Hydroxypropylmethylcellulose, Hydroxypropylcellulose, Polyvinylpyrrolidon, Polyethylenglykol und Hydroxypropylmethylcellulosephthalat 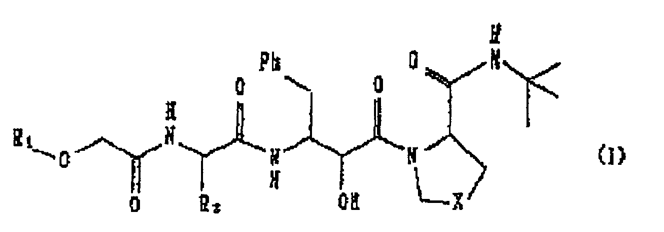
wobei R₁ eine 5-Isochinolylgruppe oder 3-Pyridylgruppe repräsentiert, R₂ eine Methylthiomethylgruppe oder Isopropylgruppe repräsentiert und X ein Schwefelatom oder eine Methylengruppe repräsentiert und wobei die feste Zusammensetzung 0,5 - 10 Gewichtsteile Bindemittel für 10 Gewichtsteile der Verbindung der Formel (I) oder deren Salz enthält.

2. Pharmazeutische Anti-AIDS-Präparate nach Anspruch 1, wobei die Kernpartikel Partikel mit einem Durchmesser zwischen 100 und 700 µm sind.

3. Pharmazeutische Anti-AIDS-Präparate nach Anspruch 1 oder 2, wobei die Kernpartikel aus kristalliner Cellulose bestehen.

4. Pharmazeutische Anti-AIDS-Präparate nach den Ansprüchen 1-3, wobei das Bindemittel Hydroxypropylmethylcellulose ist.

5. Anti-AIDS-Agens nach den Ansprüchen 1 bis 4, wobei die Verbindung der allgemeinen Formel (I) eine 3-(2S,3S)amino-4-phenylbutanoyl-1,3-thiazolin-4-carboxamid-Struktur hat.

6. Anti-AIDS-Agens nach Anspruch 5, wobei die Verbindung der allgemeinen Formel (I) (R)-N-tert-butyl-3-[(2S,3S)-2-hydroxy-3-[(R)-2-(5-isochinolyloxyacetyl)amino-3-methylthiopropanoyl] amino-4-phenylbutanoyl]-1,3-thiazolidin-4-carboxamid ist.

7. Verfahren zur Herstellung von pharmazeutischen Anti-AIDS-Präparaten, umfassend das Sprühtrocknen einer Lösung oder Suspension, die folgendes enthält:
(i) Verbindungen der allgemeinen Formel (I) oder pharmakologisch akzeptable Salze davon,
(ii) ein pharmakologisch akzeptables Bindemittel, ausgewählt aus Hydroxypropylmethylcellulose, Hydroxypropylcellulose, Polyvinylpyrrolidon, Polyethylenglykol und Hydroxypropylmethylcellulosephthalat, und
(iii) niedere Alkohole oder ein Gemisch aus niederen Alkoholen und Wasser als Lösungsmittel,
(iv) wobei die Beschichtungslösung oder -suspension 10 Gewichtsteile der Verbindung der allgemeinen Formel (I) und 0,5-10 Gewichtsteile des Bindemittels enthält,
auf pharmazeutisch akzeptable Kernpartikel, so dass die Partikel mit nichtkristallinen Formen der Verbindungen der Formel (I) oder deren Salzen und dem Bindemittel gleichmäßig beschichtet werden; und Trocknen der beschichteten Partikel.

8. Verfahren zur Herstellung pharmazeutischer Anti-AIDS-Präparate, einschließlich des Schritts der weiteren Beschichtung pharmazeutischer Präparate, die mit einem Verfahren gemäß Anspruch 7 hergestellt werden, mit einem enterischen Beschichtungsmittel.

9. Verfahren zur Herstellung pharmazeutischer Anti-AIDS-Präparate nach Anspruch 8, wobei das enterische Beschichtungsmittel Hydroxypropylmethylcellulosephthalat ist.

## Revendications

1. Préparations pharmaceutiques anti-SIDA comprenant des particules de noyau pharmaceutiquement acceptables enrobées d'une composition solide composée de
(i) un composé non cristallin de la formule (I) présentée ci-dessous ou un sel de celui-ci pharmaceutiquement acceptable et
(ii) un liant pharmaceutiquement acceptable sélectionné d'hydroxypropylméthylcellulose, d'hydroxypropylcellulose, de polyvinylpyrrolidone, de polyéthylène glycol et de phtalate d'hydroxypropylméthylcellulose. 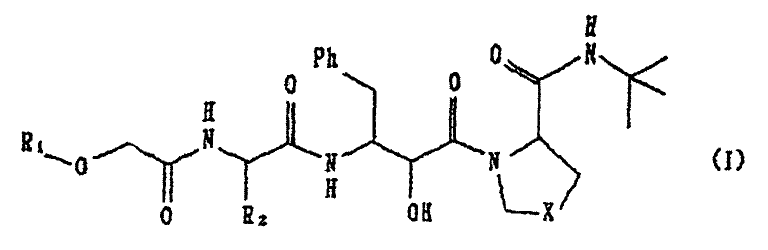
où R₁ représente un groupement isoquinolyle-5 ou un groupement pyridyle-3, R₂ représente un groupement méthylthiométhyle ou un groupement isopropyle et X représente un atome de soufre ou un groupement méthylène et où la composition solide contient 0,5-10 parties en poids de liant pour 10 parties en poids de composé de la formule (I) ou sel de celui-ci.

2. Préparations pharmaceutiques anti-SIDA selon la revendication 1, dans lesquelles les particules de noyau sont des particules ayant des diamètres de 100-700 µ m.

3. Préparations pharmaceutiques anti-SIDA selon la revendication 1 ou 2, dans lesquelles les particules de noyau sont faites de cellulose cristalline.

4. Préparations pharmaceutiques anti-SIDA selon les revendications 1-3, dans lesquelles le liant est de l'hydroxypropylméthylcellulose.

5. Agent anti-SIDA selon les revendications 1-4, dans lequel le composé présenté par la formule générale (I) a une structure 3-(2S,3S) amino-4-phénylbutanoyl-1,3-thiazoline-4-carboxamide.

6. Agent anti-SIDA selon la revendication 5, dans lequel le composé présenté par la formule générale (I) est du (R)-N-tert-butyl-3-[(2S,3S)-2-hydroxy-3-[(R)-2-(5-isoquinolyloxyacétyl) amino-3-méthylthiopropanoyl] amino-4-phénylbutanoyl]-1,3-thiazoline-4-carboxamide.

7. Procédé pour la production de préparations pharmaceutiques anti-SIDA comprenant sécher par pulvérisation une solution ou une suspension contenant
(i) des composés présentés par la formule générale (I) ou des sels pharmaceutiquement acceptables de ceux-ci,
(ii) un liant pharmaceutiquement acceptable sélectionné d'hydroxypropylméthylcellulose, d'hydroxypropylcellulose, de polyvinylpyrrolidone, de polyéthylène glycol et de phtalate d'hydroxypropylméthylcellulose et
(iii) des alcools inférieurs ou un mélange d'alcools inférieurs/eau comme solvant
(iv) où la solution ou suspension d'enrobage contient 10 parties en poids du composé présenté par la formule générale (I) et 0,5-10 parties en poids du liant,
sur des particules de noyau pharmaceutiquement acceptables de sorte que les particules deviennent enrobées uniformément de formes non cristallines des composés de la formule (I) ou sels de ceux-ci et le liant; et sécher les particules enrobées.

8. Procédé pour la production de préparations pharmaceutiques anti-SIDA incluant l'étape consistant à enrober encore les préparations pharmaceutiques préparées par un procédé selon la revendication 7 avec un agent d'enrobage entérique.

9. Procédé pour la production de préparations pharmaceutiquement acceptables selon la revendication 8, dans lequel l'agent d'enrobage entérique est du phtalate d'hydroxypropylméthylcellulose.
